# EUROPEAN PATENT SPECIFICATION

(11) **EP 3 656 345 B1**
(45) Date of publication and mention of the grant of the patent: **11.08.2021**
(21) Application number: 20150144.2
(22) Date of filing: 04.11.2016
(51) Int. Cl.: A61C 7/08, A61B 5/01, A61B 5/022, A61B 5/024, A61B 5/08, A61B 5/145, A61B 5/00

(54) **WEARABLE APPARATUS ATTACHING ON TOOTH AND THE SENSING DEVICE FIXING AT TOOTH**
AM ZAHN ZU BEFESTIGENDE TRAGBARE VORRICHTUNG UND DIE AM ZAHN ZU BEFESTIGENDE MESSVORRICHTUNG
APPAREIL VESTIMENTAIRE S'ATTACHANT À UNE DENT ET DISPOSITIF DE DÉTECTION SE FIXANT À UNE DENT

(43) Date of publication of application: 27.05.2020
(62) Divisional of application: 16197274.0
(73) Proprietor: Lee, Jin Kyun, Seoul 07020 (KR)
(72) Inventor: Lee, Jin Kyun, Seoul 07020 (KR)
(74) Representative: Berggren Oy

(56) References cited:
- US-A1- 2006 166 157
- US-A1- 2012 172 677
- US-A1- 2013 109 932

## Description

### TECHNICAL FIELD

At least one example embodiment relates to a tooth-attach wearable device and a tooth-fix sensing device, and more particularly, to a tooth-attach wearable device that may verify whether a tooth-attach wearable device, such as a transparent orthodontic device, is worn by a patient, that is, attached to teeth of the patient and an amount of time in which the tooth-attach wearable device is worn, that is, attached, may sense biometric information of the patient from the saliva of the patient, may store the sensed biometric information as data, and may remotely transmit the stored data for monitoring, and a tooth-fix sensing device that may attach a sensor for sensing the saliva or a temperature of a patient to a device, such as a plastic orthodontic bracket, may store information about the sensed saliva or temperature as data, and may remotely transmit the stored data for monitoring.

### RELATED ART

In general, types of an orthodontic device include a metal orthodontic device, a ceramic orthodontic device, a modified tandem appliance (MTA) orthodontic device, a clippy-C orthodontic device, a Damon orthodontic device, and the like.

Currently, many patients select a transparent orthodontic device among such various orthodontic devices. As for the greatest advantage, the transparent orthodontic device has an aesthetic property. That is, a patient may not appear to wear the orthodontic device. In many cases, an orthodontic treatment may be performed without tooth extraction. The patient may take out or insert the orthodontic device in person and may experience a relatively less pain compared to other wire orthodontic devices during an orthodontic treatment process.

However, since the patient may take out or insert the orthodontic device in person, the patient may have a degraded effect and experience an increased treatment term if the patient does not wear the orthodontic device in an appropriate manner.

Document US 2006/166157 A1 presents an orthodontic compliance monitor including a sensor that senses when an orthodontic appliance is properly positioned and a processor that processes an output of the sensor and generates compliance data. A memory device stores compliance data and the processor generates the compliance data based on a compliance protocol. System and methods for orthodontic compliance are also presented.

### DETAILED DESCRIPTION

An objective of the present invention is to provide a tooth-attach wearable device. The objective is reached by a tooth-attach wearable device as defined by the independent claim 1. Preferred embodiments of the invention are defined in the dependent claims.

### BRIEF DESCRIPTION OF DRAWINGS

FIG. 1 is a perspective view illustrating a tooth-attach wearable device according to the invention.
FIG. 2 is block diagram illustrating a tooth-attach wearable device according to the invention.
FIG. 3 is a view illustrating a thermoelement provided to a sensor head of a lower tooth-attach wearable device according to the an embodiment, which does not form part of the invention.
FIG. 4 is a view illustrating a chemical substance detection element provided to a sensor head of a lower tooth-attach wearable device according to an embodiment, which does not form part of the invention.
FIG. 5 is a view illustrating a piezoelectric element provided to a sensor head of a lower tooth-attach wearable device according to an embodiment, which does not form part of the invention.
FIG. 6 is a block diagram illustrating a vibration sensor provided to a tooth-attach wearable device according to an embodiment, which does not form part of the invention.
FIG. 7 is a view illustrating a touch sensor provided to a lower tooth-attach wearable device according to an embodiment, which does not form part of the invention.
FIG. 8 is a view illustrating a tooth-attach wearable device attached to a portion of teeth of a patient according to the invention.
FIG. 9 is a view illustrating a tooth-fix sensing device according to the invention.
FIG. 10 is a view illustrating a tooth-fix sensing device observed from side according to an embodiment, which does not form part of the invention.
FIG. 11 is a view illustrating a tooth-fix sensing device according to an embodiment, which does not form part of the invention.
FIG. 12 illustrates an example of remotely transferring biometric information of a patient using a communication controller of a tooth-attach wearable device according to the invention.

### DETAILED DESCRIPTION TO CARRY OUT THE DISCLOSURE

Hereinafter, example embodiments will be described with reference to the accompanying drawings.

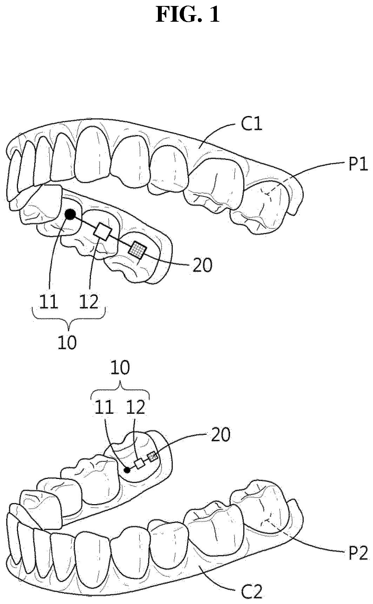

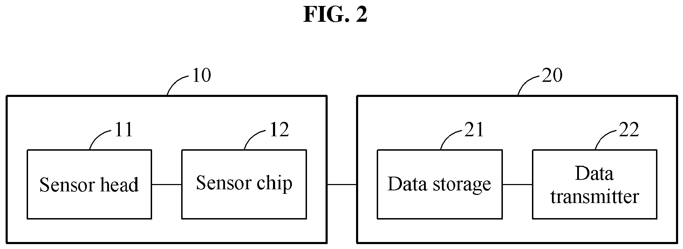

FIG. 1 is a perspective view illustrating a tooth-attach wearable device according to a first example embodiment, and FIG. 2 is a block diagram illustrating a tooth-attach wearable device according to the first example embodiment.

Hereinafter, a description will be made based on an example in which a tooth-attach wearable device C1, C2 according to an example embodiment is a transparent orthodontic device. Although the description is described herein based on the example in which the tooth-attach wearable device C1, C2 is a transparent orthodontic device, the present disclosure is not limited to a transparent material or an orthodontic device. Thus, any type of devices detachably attached to teeth may be applicable.

The transparent orthodontic device may be formed using polyethylene terephthalate (PET) or a specially reinforced plastic material. The specially reinforced plastic material has some advantages, such as a relatively great strength, a significant lightness, and a small abrasion compared to a general plastic. Thus, the specially reinforced plastic material does not add difficulty to teeth and is not easily deformed even after long use. In addition, if the specially reinforced plastic material is formed using a transparent specially reinforced plastic, an aesthetic property is excellent in that a patient may not appear to wear the orthodontic device although the patient wears the orthodontic device.

Referring to FIGS. 1 and 2, the tooth-attach wearable device C1, C2 includes a sensor device 10 and a communication controller 20. In more detail, the tooth-attach wearable device C1, C2 may be divided into an upper tooth-attach wearable device C1 and a lower tooth-attach wearable device C2.

The sensor device 10 refers to a portion that becomes into contact with a tongue or gums of the patient, and may be exposed outside a tooth attachment P1, P2 formed on the tooth-attach wearable device C1, C2.

The sensor device 10 includes a sensor head 11 configured to sense biometric information of the patient by sensing the saliva or a temperature of the patient, and a sensor chip 12 configured to couple with the sensor head 11. One of a thermoelement 11a, a piezoelectric element 11b, a chemical substance detection element 11c, and a marker sensor (not shown) may be provided to the sensor head 11. However, the example embodiments are not limited thereto.

In particular, the sensor head 11 included in the sensor device 10 is a portion that is exposed outside the tooth attachment P1, P2. Thus, a portion of the sensor head 11 may be coated with a hydrophobic coating film not be deformed by the saliva of the patient, etc. On the contrary, the sensor chip 12 and the communication controller 20, which will be described below, are embedded in the tooth attachment P1, P2 and thereby coated and thus, may be completely prevented from the saliva.

The communication controller 20 includes a data storage 21 configured to store the biometric information sensed at the sensor device 10 as data, and a data transmitter 22 configured to remotely transmit the data stored in the data storage 21. Here, a monitoring device M, M' configured to display the data transmitted from the data transmitter 22 may be further included. For example, the monitoring device M, M' may refer to a desktop personal computer (PC), a laptop computer, a smartphone, and other various types of display devices capable of performing remote transmission. The above various types of display devices capable of performing remote transmission may be any type of objects on which images, letters, numbers, etc., can be displayed. For example, a window, a front glass window of a vehicle, etc., may be included in the various types of display devices capable of performing remote transmission.

The communication controller 20 may couple with the tooth-attach wearable device C1, C2 by being internally molded therein.

Meanwhile, referring to FIG. 1, the sensor device 10 and the communication controller 20 may be mounted across a plurality of teeth and may be mounted to a single tooth.

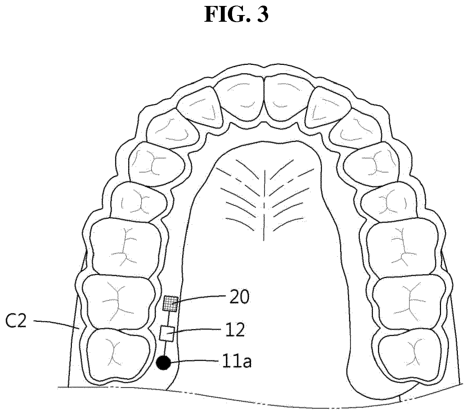

FIG. 3 is a view illustrating a thermoelement provided to a sensor head of a lower tooth-attach wearable device according to the first example embodiment.

Referring to FIG. 3, the thermoelement 11a is provided to the sensor head 11 of the lower tooth-attach wearable device C2 according to the example embodiment.

In detail, the sensor device 10 senses whether the tooth-attach wearable device C1, C2 is attached to teeth by sensing a temperature of the patient. Once the tooth-attach wearable device C1, C2 is sensed to be attached, the sensor device 10 may measure an amount of time in which the tooth-attach wearable device C1, C2 is attached to the teeth. If the tooth-attach wearable device C1, C2 is a transparent orthodontic device, the sensor device 10 or the communication controller 20 may include a time measurer (not shown) configured to record an amount of time from a time at which the tooth-attach wearable device C1, C2 is attached to the teeth to a time at which the tooth-attach wearable device C1, C2 is detached (separate) from the teeth. The time measurer may be set to measure and accumulate an amount of time only in response to receiving electricity being supplied from the thermoelement 11a. The thermoelement 11a is set to generate electricity and to supply the electricity to the time measurer only when the temperature measured at the sensor device 10 is between about 35°C and about 38°C, which is close to a general human temperature. A measured temperature range such that the thermoelement 11a generates the electricity may be appropriately modified based on a situation of a user, for example, the patient.

In the above manner, an amount of time in which the transparent orthodontic device is attached to the teeth may be measured. Since the patient is allowed to freely put on or take out the transparent orthodontic device, it is important for the patient to voluntarily wear the orthodontic device in order to enhance the orthodontic effect. The transparent orthodontic device may be applicable to any age range except for a child of which physique basically varies. However, when the patient voluntarily wears the orthodontic device for 17 hours or more, the orthodontic effect may be acquired. For example, if the average amount of time in which the patient wears the orthodontic device is measured at the time measurer to be less than 17 hours a day, it may be determined that the orthodontic treatment period may increase.

Accordingly, the sensor device 10 may sense that the patient puts on, that is, attaches the tooth-attach wearable device C1, C2 to teeth, and may provide information used to determine the orthodontic period and the orthodontic state of the patient.

The thermoelement 11a is exposed outside the tooth attachment P2 and may directly contact with the saliva of the patient. Here, a portion of the sensor device 10 may be coated with a hydrophobic coating film (not shown) to prevent deformation or malfunction by the saliva. The hydrophobic coating film directly contacts with a tooth and thus, may be formed using a material that may not damage the tooth.

A wirelessly chargeable battery (not shown) may be provided to the sensor chip 12, and electricity stored in the battery may be supplied to the sensor head 11. The sensor chip 12 may be wirelessly charged from the wireless charging device in a form of an ear ring, a headset, or Bluetooth that couples with an ear in a state in which the tooth-attach wearable device C1, C2 is attached to the teeth of the patient. Alternatively, if the tooth-attach wearable device C1, C2 is not used, the battery of the sensor chip 12 may be charged by placing the tooth-attach wearable device C1, C2 on the wireless charging device.

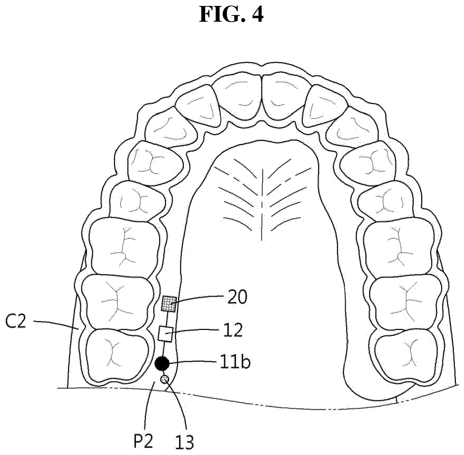

FIG. 4 is a view illustrating a chemical substance detection element provided to a sensor head of a lower tooth-attach wearable device according to a second example embodiment.

Referring to FIG. 4, the lower tooth-attach wearable device C2 according to the second example embodiment may include the sensor head 11, the sensor chip 12, a saliva container 13, and the communication controller 20. Here, the chemical substance detection element 11b may be provided to the sensor head 11.

The sensor head 11 may couple with one surface of the tooth attachment P2, and may be exposed inside the oral cavity, and may sense the saliva of the patient. In more detail, the chemical substance detection element 11b may sense glucose among a plurality of components included in the saliva and may measure blood sugar of the patient. In addition, the chemical substance detection element 11b may measure amounts or types of various hormones, such as a cortisol hormone, a change therein, and the like, and may detect the presence or absence of various types of diseases.

Acquiring biometric information of the patient from the saliva of the patient may be a new method capable of replacing a standard blood test. The saliva generally includes about 99 % of moisture, whereas important biometric information may be acquired from remaining 1% of the saliva. In addition to biological information and genetic information, materials helpful to fight against the diseases may be acquired from the saliva. In particular, various types of biometric information may be easily acquired using the saliva. For example, a specific protein used as a cardiac risk index in a blood test may be acquired from the saliva. Thus, such a saliva test may replace the blood test.

The saliva may be positioned in a lower portion of the oral cavity due to gravity. Thus, the sensor head 11 for sensing the saliva may be provided to the lower tooth-attach wearable device C2.

The saliva sensed at the sensor head 11 may be spit and may also be a component, such as a gingival crevicular fluid secreted between teeth and gums. The gingival crevicular fluid refers to the saliva discharged from above a boundary between the teeth and the gingiva, and may have a relatively high medical accuracy in the aforementioned hormone related measurement compared to the spit.

Meanwhile, the chemical substance detection element 11b may measure bad breadth inside the oral cavity. The chemical substance detection element 11b may employ any known configuration to measure the bad breadth. The chemical substance detection element 11b for measuring the bead breadth may measure a level of bad breadth based on the saliva inside the oral cavity or the air inside the oral cavity.

The chemical substance detection element 11b may be positioned at a location corresponding to an innermost tooth attachment P2 of the lower tooth-attach wearable device C2, that is, the tooth attachment P2 in contact with an innermost molar since the parotid gland and the submandibular gland are largest among salivary glands of a human being and the parotid gland and the submandibular gland are positioned to be closest to molars among teeth. The stress may increase adrenaline and the secretion of the saliva, and may make a heart beat quickly. Here, an enzyme, such as alpha-amylase, is secreted in the salivary gland. A stress diagnosis may be performed through cortisol hormone measurement, and may be used as an index of stress.

The sensor chip 12 may couple with the sensor head 11, and may be fixed to the lower tooth attachment P2 and supply the electricity to the sensor head 11. Here, a wirelessly chargeable battery may be provided to the sensor chip 12, and electricity stored in the battery may be supplied to the sensor head 11. The sensor chip 12 may be wirelessly charged from the wireless charging device in a form of an ear ring, a headset, or Bluetooth that couples with an ear in a state in which the lower tooth-attach wearable device C2 is attached to the teeth of the user. Alternatively, if the lower tooth-attach wearable device C2 is not used, the battery of the sensor chip 12 may be charged by placing the lower tooth-attach wearable device C2 on the wireless charging device.

At least one saliva container 13 may be formed on the tooth attachment P2, and may be formed in a shape, such as a bowl, capable of containing the saliva of the patient. The saliva container 13 may be provided at a location adjacent to the sensor head 11 so that the sensor head 11 may sense the saliva of the patient contained in the saliva container 13.

In addition to the blood sugar, the sensor head 11 may also measure biometric information, such as blood pressure, electrocardiogram (ECG), and pulse. The sensor head 11 for measuring the blood pressure or ECG may not be exposed from the tooth attachment P1, P2. In detail, the sensor head 11 for measuring the blood pressure or the ECG may not be exposed from the tooth attachment P1, P2 toward an inside of the oral cavity, and may measure the blood pressure or the ECG in contact with the gums, etc. A known blood pressure or ECG measurement device may be provided to the sensor head 11 and may measure the blood pressure or the ECG. The sensor head 11 for measuring biometric information, such as blood pressure or ECG may not be necessarily provided to the lower tooth-attach wearable device C2, and may be provided to the upper tooth-attach wearable device C1.

Meanwhile, a marker sensor capable of sensing a bio-marker and the like may be provided to the sensor head 11. The marker sensor may verify various types of hormone indices by verifying a specific genetic trait included in the saliva and may diagnose various types of diseases accordingly.

The communication controller 20 may include the data storage 21 configured to store the saliva information sensed at the sensor head 11 as data and the data transmitter 22 configured to remotely transmit the data stored in the data storage 21. Here, the monitoring device M, M' configured to display the data transmitted from the data transmitter 22 may be further included. For example, the monitoring device M, M' may be a desktop PC, a laptop computer, a smartphone, and any type of display devices capable of performing remote transmission.

The communication controller 20 may couple with the tooth-attach wearable device C1, C2 by being internally molded therein.

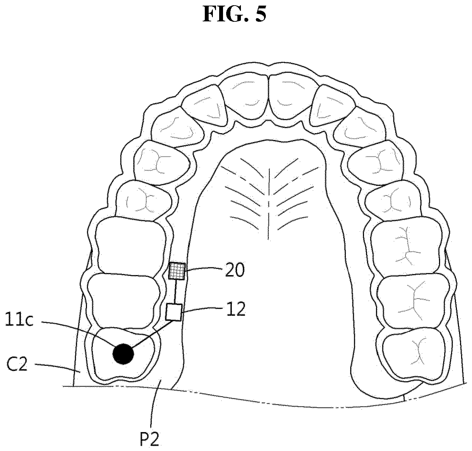

FIG. 5 is a view illustrating a piezoelectric element provided to a sensor head of a lower tooth-attach wearable device according to a third example embodiment.

Referring to FIG. 5, the lower tooth-attach wearable device C2 according to the third example embodiment may include the sensor head 11, the sensor chip 12, and the communication controller 20. The piezoelectric element 11c may be provided to the sensor head 11.

The sensor head 11 may couple with one surface of the tooth attachment P2, that is, an engagement surface, and may sense an engagement force between a tooth and the tooth attachment P2. Here, the sensor head 11 may be provided on the engagement surface of the tooth attachment P2 that faces a top surface of the tooth of the patient. If the patient puts on the lower tooth-attach wearable device C2 at the lower teeth, the sensor head 11 may sense a force occurring when the lower teeth and the upper teeth are engaged in response to shutting the patient's mouth. Accordingly, the sensor head 11 may sense the engagement force between the tooth and the tooth attachment P2, may determine whether bruxism of the patient is present, may measure the engagement force between the upper teeth and the lower teeth, and may verify the effect of measured engagement force against the teeth.

In detail, since an amount of electricity generated at the piezoelectric element 11c increases according to an increase in pressure applied to the sensor head 11, the piezoelectric element 11c provided to the sensor head 11 may determine whether bruxism of the patient is present and may measure the engagement force between the upper teeth and the lower teeth by measuring the electricity generated at the piezoelectric element 11c.

Also, since the engagement force occurring when the patient shuts the patient's mouth is sensed at the sensor head 11, the sensor chip 12 and the communication controller 20 may be provided to the side of the tooth attachment P2 instead of being provided to the top surface of the tooth attachment P2.

Once the piezoelectric element 11c is provided to the sensor head 11, it is possible to determine a temporomandibular disorder of the patient. The temporomandibular disorder may be solved by employing an in-mouth device in a form of orthodontic treatment or mouthpiece. The mouthpiece may be applicable to treat the temporomandibular disorder by applying principles of the tooth-attach wearable device C1, C2 to the mouthpiece.

The sensor chip 12 may couple with the sensor head 11, and may be fixed to the lower tooth attachment P2 and supply electricity to the sensor head 11. Here, a wirelessly chargeable battery may be provided to the sensor chip 12, and electricity stored in the battery may be supplied to the sensor head 11. The sensor chip 12 may be wirelessly charged from the wireless charging device in a form of an ear ring, a headset, or Bluetooth that couples with an ear in a state in which the lower tooth-attach wearable device C2 is attached to the teeth of the user. Alternatively, if the lower tooth-attach wearable device C2 is not used, the battery of the sensor chip 12 may be charged by placing the lower tooth-attach wearable device C2 on the wireless charging device.

Alternatively, electric energy generated at the piezoelectric element 11c with chewing pressure energy of the user may be stored in the battery. Such energy stored in the battery may be used to drive the sensor device 10 and the communication controller 20, etc. Accordingly, using the electric energy generated when a pressure is applied to the piezoelectric element 11c, the piezoelectric element 11c may determine whether bruxism of the patient is present and may measure the engagement force between the upper teeth and the lower teeth. Alternatively, the piezoelectric element 11c may store the electric energy and may provide the electric energy to other constituent elements, such as the sensor device 10 and the communication controller 20, etc.

The communication controller 20 may include the data storage 21 configured to store magnitude information of the engagement force between the tooth and the tooth attachment P2 sensed at the sensor head 11 as data and the data transmitter 22 configured to remotely transmit the data stored in the data storage 21. Here, the monitoring device M, M' configured to display the data transmitted from the data transmitter 22 may be further included. For example, the monitoring device M, M' may be a desktop PC, a laptop computer, a smartphone, and any type of display devices capable of performing remote transmission

The communication controller 20 may couple with the tooth-attach wearable device C1, C2 by being internally molded therein.

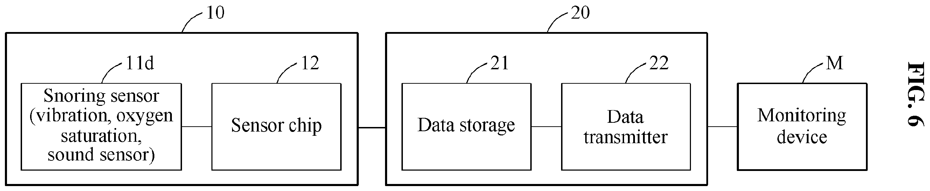

FIG. 6 is a block diagram illustrating a vibration sensor provided to a tooth-attach wearable device according to a fourth example embodiment.

Referring to FIG. 6, the tooth-attach wearable device C1, C2 according to the fourth example embodiment may include a vibration sensor 11d, the sensor chip 12, the communication controller 20, and the monitoring device M.

The vibration sensor 11d may couple with one surface of the tooth attachment P1, P2 and may sense a vibration by snoring. In more detail, the vibration sensor 11d may sense a vibration by snoring occurring between a nose and vocal chords. In general, air passages may become narrow and air may flow when the user breathes. In this instance, snoring may occur while causing friction, a vibration, etc., in uvula or plate of the user. Accordingly, the vibration sensor 11d may sense a vibration occurring when the air flows through air passages and may sense the occurrence of snoring during a sleep.

The sensor chip 12 may couple with the vibration sensor 11d, and may be fixed to the tooth attachment P1, P2 and supply electricity to the vibration sensor 11d.

The communication controller 20 may couple with the sensor chip 12, and may be fixed to the tooth attachment P1, P2.

The communication controller 20 may store vibration information sensed at the vibration sensor 11d and may serve to transmit the stored vibration information.

The communication controller 20 may include the data storage 21 configured to store the vibration information sensed at the vibration sensor 11d as data and the data transmitter 22 configured to remotely transmit the data stored in the data storage 21. Here, the monitoring device M configured to display the data transmitted from the data transmitter 22 may be included.

That is, the vibration sensor 11d may determine the presence or absence of snoring by sensing a vibration by snoring occurring between the nose and the vocal chords, and by displaying the transmitted vibration information on the monitoring device M.

Also, in addition to the method of sensing a vibration by snoring, it is possible to verify an oxygen saturation occurring when the patient breathes or a sound occurring when the patient snores. In the case of sensing the oxygen saturation, an oxygen saturation sensor may be used instead of the vibration sensor. In the case of sensing the sound, a sound sensor may be used instead of the vibration sensor.

If the oxygen saturation is less than an average value, the oxygen saturation may be used as an index to determine sleep disorders, such as snoring, obstructive sleep apnea, etc., occurring due to lack of oxygen, and diseases, such as a stroke, heart attack, etc. The saturation of oxygen sensed inside the oral cavity may decrease when the user snores compared to a case in which the user does not snore. Thus, it is possible to determine the presence or absence of snoring and to monitor a level of snoring in real time by sensing the oxygen saturation.

Also, when the user snores, a snoring sound occurs. Thus, it is possible to monitor the occurrence of sound and a change in a magnitude of sound in real time. The oxygen saturation and sound information sensed at the oxygen saturation sensor and the sound sensor may be transmitted to the monitoring device M, and may be used to determine the presence or absence of snoring and a level of snoring.

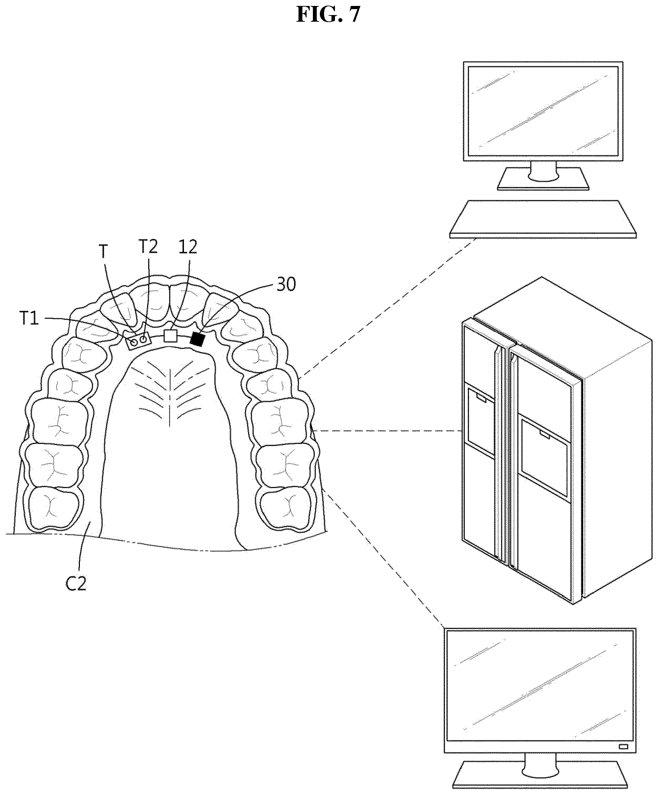

FIG. 7 is a view illustrating a touch sensor provided to a lower tooth-attach wearable device according to a fifth example embodiment.

Referring to FIG. 7, the lower tooth-attach wearable device C2 according to the fifth example embodiment may include a touch sensor T, the sensor chip 12, and a transmitter 30.

The touch sensor T may couple with one surface of the tooth attachment P2, and at least one touch panel T1, T2 may be provided.

For example, the touch sensor T may be provided in a structure similar to a mouth that includes at least two touch panels T1 and T2. In response to pushing one of the two touch panels T1 and T2, the transmitter 30 may transmit a signal to a computer, a TV, a refrigerator, a washing machine, a boiler, etc.

The sensor chip 12 may couple with the touch sensor T, and may be fixed to the tooth attachment P2 and supply electricity to the touch sensor T.

The transmitter 30 may couple with the sensor chip 12, may be fixed to the tooth attachment P2, and may transmit a signal to a computer and the like in response to pushing the touch panel T1, T2.

For example, the touch sensor T may contact with the tongue of the user and thus, may be positioned in an inner side of the middle of the lower tooth-attach wearable device C2 as shown in FIG. 7.

The user wearing the lower tooth-attach wearable device C2 according to the example embodiment may click a cursor of a computer by pushing the touch panel T1, T2 with the tongue of the user, and may also move the cursor on a monitor. The user wearing the lower tooth-attach wearable device C2 may power on a TV, a refrigerator, a washing machine, a boiler, etc., by pushing the touch panel T1, T2 with the tongue, or may transfer a signal to such devices and may manipulate the devices. For example, by pushing the touch panel T1, T2 with the tongue, the user wearing the lower tooth-attach wearable device C2 may power on a TV or select a channel, may power on a refrigerator or adjust an inside temperature of the refrigerator, and may power on a boiler or adjust an operating temperature of the boiler.

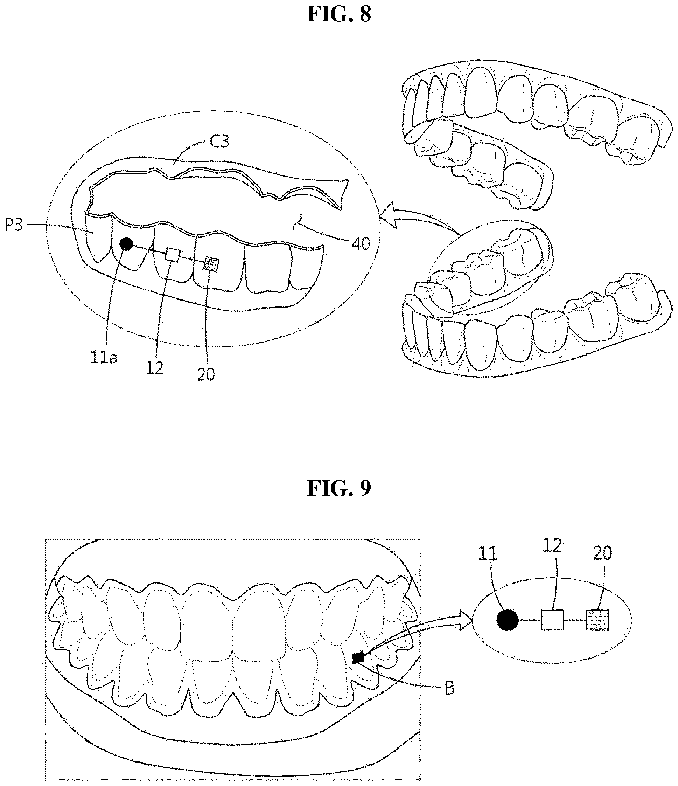

FIG. 8 is a view illustrating a tooth-attach wearable device attached to a portion of teeth of a patient according to a sixth example embodiment.

Referring to FIG. 8, a tooth-attach wearable device C3 according to the sixth example embodiment may include the sensor device 10 and the communication controller 20.

Also, a cutting line 40 of which one surface is cut may be formed on the tooth-attach wearable device according to the sixth example embodiment.

The thermoelement 11a or the chemical substance detection element 11b may be provided to the sensor head 11 of the sensor device 10. In this instance, the piezoelectric element 11c may not be provided to the sensor head 11 since an engagement force between a tooth and the tooth attachment P2 may not be measured due to the cutting line 40 formed on the tooth-attach wearable device.

The sensor head 11 may couple with one surface of a tooth attachment P3, and may be exposed inside the oral cavity and may sense the saliva or a temperature of the patient.

The sensor chip 12 may couple with the sensor head 11, and may be fixed to the tooth attachment P3 and supply electricity to the sensor head 11.

The communication controller 20 may couple with the sensor chip 12 and may be fixed to the tooth attachment P3. The communication controller 20 may store saliva information or temperature information sensed at the sensor head 11 and may transmit the stored saliva information or temperature information.

The tooth-attach wearable device C3 may be attached only to a desired tooth instead of being applied to all of the teeth. For example, referring to FIG. 8, the tooth-attach wearable device C3 may be attached to a molar portion. Without being limited to the size and the shape of FIG. 8, the tooth-attach wearable device C3 may be manufactured in various types.

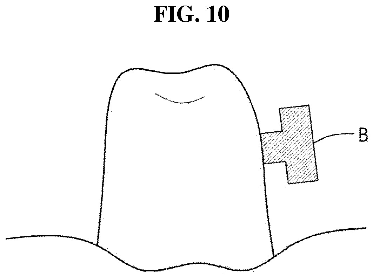

FIG. 9 is a view illustrating a tooth-fix sensing device according to a seventh example embodiment, and FIG. 10 is a view illustrating a tooth-fix sensing device observed from side according to the seventh example embodiment.

Referring to FIGS. 9 and 10, the tooth-fix sensing device according to the seventh example embodiment may be a plastic orthodontic bracket B fixed to a tooth (teeth).

The sensor head 11, the sensor chip 12, and the communication controller 20, etc., provided to the aforementioned tooth-attach wearable device C1, C2, C3 may also be applied to the plastic orthodontic bracket B in the same manner.

Here, since the plastic orthodontic bracket B is not detachably attached like the transparent orthodontic device, there is no need to sense a temperature of the user and to measure an amount of time in which the plastic orthodontic bracket B is attached to the teeth.

The tooth-fix sensing device may include the orthodontic bracket B provided to at least a portion of teeth, the sensor head 11 provided to the orthodontic bracket B and exposed inside the oral cavity to sense biometric information in contact with the saliva of the patient, the sensor chip 12 configured to couple with the sensor head 11 and provided to the orthodontic bracket B to supply electricity to the sensor head 11, and the communication controller 20 configured to couple with the sensor chip 12 and provided to the orthodontic bracket B to store the biometric information sensed at the sensor head 12 and to transmit the stored biometric information.

Here, a chemical substance detection element may be provided to the sensor head 11 that is provided to the orthodontic bracket B. The chemical substance detection element may sense glucose in the saliva and may measure blood sugar of the patient or may measure biometric information such as amounts or types of hormones, change therein, bad breathing, blood pressure, ECG, and pulse. Similar to other tooth-attach wearable devices C1, C2, C3, all of the sensor head 11, the sensor chip 12, and the communication controller 20 may be provided to a single plastic orthodontic bracket B.

The sensor head 11 may be exposed from the plastic orthodontic bracket B or may be embedded therein based on a type of an element provided to the sensor head 11. For example, in the case of sensing biometric information from the saliva of the patient, the sensor head 11 may be exposed inside the oral cavity, and the chemical substance detection element 11b may be provided to the sensor head 11.

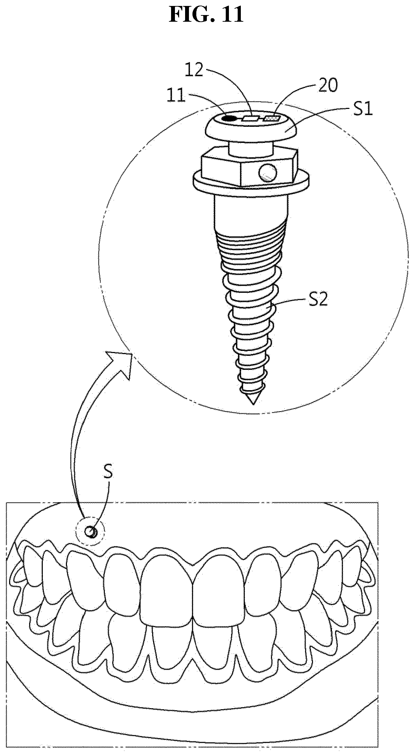

FIG. 11 is a view illustrating a tooth-fix sensing device according to an eighth example embodiment.

Referring to FIG. 11, the tooth-fix sensing device according to the eighth example embodiment may be a mini screw S that is connected to a plastic orthodontic bracket (not shown) using an elastic band and prevents a tooth being corrected from moving in an undesired direction.

The mini screw S may be fixed to the alveolar bone by passing through the gums, that is, periodontal tissue of the patient. The mini screw S may include a mini screw head S1 configured to be exposed outside the periodontal tissue and a mini screw body S2 configured to insert into the periodontal tissue. The mini screw head S1 may detachably couple with the mini screw body S2.

In more detail, the sensor head 11, the sensor chip 12, and the communication controller 20 may be provided to the mini screw head S1. The sensor head 11 may couple with the mini screw head S1, and may be exposed inside the oral cavity and sense the saliva. Accordingly, the sensor head 11 according to the eighth example embodiment may be provided as the chemical substance detection element 11b.

Also, although FIG. 11 illustrates that the sensor chip 12 and the communication controller 20 are included in the mini screw head S1 and are exposed inside the oral cavity, they except for the sensor head 11 for sensing biometric information from the saliva of the patient may be provided with being embedded in the mini screw head S1

The chemical substance detection element may be provided to the sensor head 11 that is provided to the mini screw head S1. The chemical substance detection element may sense glucose in the saliva and may measure blood sugar of the patient or may measure biometric information, such as amounts or types of hormones, a change therein, bad breath, blood pressure, ECG, and pulse.

The sensor chip 12 may couple with the sensor head 11, and may be fixed to the mini screw S and supply electricity to the sensor head 11.

The communication controller 20 may couple with the sensor chip 12, and may be fixed to the mini screw S, and may store the biometric information sensed at the sensor head 11 and may transmit the stored biometric information.

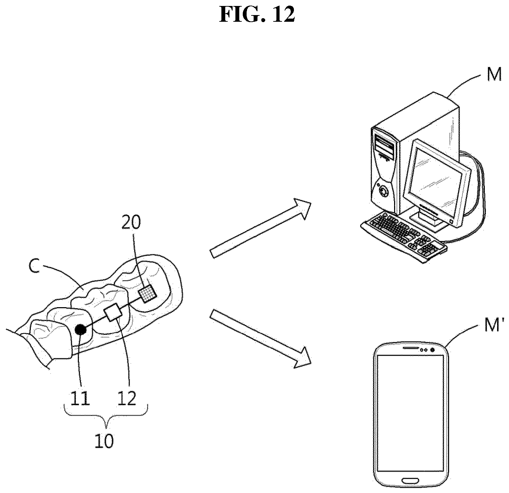

FIG. 12 illustrates an example of remotely transferring biometric information of a patient from a communication controller of a tooth-attach wearable device according to the first example embodiment.

Referring to FIG. 12, a tooth-attach wearable device C according to example embodiments may include the monitoring device M, M' configured to display data transmitted from the data transmitter 22.

The monitoring device M, M' may be, for example, a desktop PC M or a smartphone M'. Each of the desktop PC M and the smartphone M' may receive biometric information of the patient remotely transmitted from the data transmitter 22 provided to the tooth-attach wearable device C according to the example embodiments as well as the upper tooth-attach wearable device C1 and the lower tooth-attach wearable device C2.

It is possible to separately verify an amount of time in which the tooth-attach wearable device C is attached with respect to each of the upper teeth and the lower teeth using the monitoring device M, M'. Also, it is possible to analyze information obtained from the saliva and to use the analyzed information as various indices.

Hereinafter, an operation of the tooth-attach wearable device C according to the example embodiments will be described.

The tooth-attach wearable device C may include the sensor device 10 and the communication controller 20.

One of the thermoelement 11a, the chemical substance detection element 11b, and the piezoelectric element 11c may be provided to the sensor head 11 of the sensor device 10.

Initially, in an example in which the thermoelement 11a is provided to the sensor head 11, the sensor head 11 may sense a temperature of the patient at a location at which the sensor head 11 is exposed inside the oral cavity. For example, if the tooth-attach wearable device C is a transparent orthodontic device, the sensor head 11 may measure an amount of time in which the tooth-attach wearable device C is attached to the teeth by measuring the temperature of the patient, may check the measured amount of time in real time, and may use the checked amount of time to determine a subsequent orthodontic treatment direction. If the patient wears the transparent orthodontic device for less than a preset period of time, it is possible to inform the patient about an additional amount of time in which the patient is to wear the transparent orthodontic device.

In an example in which the chemical substance detection element 11b is provided to the sensor head 11, the sensor head 11 may sense the saliva of the patient at a location at which the sensor head 11 is exposed inside the oral cavity. In particular, the sensor head 11 may measure blood sugar of the patient by sensing glucose among components contained in the saliva of the patient.

In an example in which the piezoelectric element 11c is provided to the sensor head 11, the sensor head 11 may couple with a surface of the tooth attachment P1, P2 that faces a top surface of a tooth. Accordingly, the sensor head 11 may measure a magnitude of an engagement force between the tooth and the tooth attachment P1, P2 and may verify whether bruxism of the patient is present, etc.

The sensor chip 12 may couple with the sensor head 11. A wirelessly chargeable battery may be provided to the sensor chip 12, and may supply electricity stored in the battery to the sensor head 11.

The communication controller 20 may include the data storage 21 configured to store temperature information of the patient sensed at the sensor head 11 as data, and the data transmitter 22 configured to remotely transmit the data stored in the data storage 21. Here, the monitoring device M, M' configured to display the data transmitted from the data transmitter 22 may be further included.

Therefore, the tooth-attach wearable device C according to the example embodiments may monitor biometric information of the patient, and may use the biometric information as an index associated with an orthodontic state if the tooth-attach wearable device C is a transparent orthodontic device.

The tooth-attach wearable device C according to the example embodiment may be applicable to persons of various professions, such as an athlete, a soldier, etc.

## Claims

1. A tooth-attach wearable device (C, C1, C2, C3) having a tooth attachment (P1, P2, P3) corresponding to a shape of a tooth, the wearable device (C, C1, C2, C3) comprising:
a sensor head (11) configured to couple with one surface of the tooth attachment (P1, P2, P3), to be exposed inside the oral cavity, and to sense a temperature of a patient;
by wherein a time measurer configured to measure an amount of time in which the tooth attachment (P1, P2, P3) is attached to the tooth, based on the temperature sensed at the sensor head (11);
wherein a thermoelement (11a) is provided to the sensor head (11), the thermoelement (11a) generates electricity and supplies the generated electricity to the time measurer only when the temperature sensed at the sensor head (11) is between 35 °C and 38 °C;
wherein the time measurer measures and accumulates the amount of time only in response to receiving the electricity being supplied from the thermoelement (11a); and
wherein the wearable device (C, C1, C2, C3) further comprises a sensor chip (12) configured to couple with the sensor head (11), to fix to the tooth attachment (P1, P2, P3), and to supply electricity to the sensor head (11),
wherein the wearable device (C, C1, C2, C3) comprises a communication controller (20) provided to the tooth attachment (P1, P2, P3), and configured to store temperature information of the patient sensed at the sensor head (11), and to transmit the stored temperature information,
wherein the communication controller comprises a data storage (21) configured to store the temperature information of the patient sensed at the sensor head (11) as data; and a data transmitter (22) configured to remotely transmit the data stored in the data storage (21),
the wearable device (C, C1, C2, C3) further comprising: a snoring sensor configured to couple with one surface of the tooth attachment (P1, P2, P3), and to sense at least one of a vibration, an oxygen saturation, and sound by snoring,
wherein the sensor chip (12) is configured to couple with the snoring sensor, and to supply electricity to the snoring sensor; and the communication controller is configured to store at least one of a vibration, an oxygen saturation occurring in breathing, and a snoring sound sensed at the snoring sensor, and to store the sensed at least one of a vibration, an oxygen saturation occurring in breathing, and a snoring sound.

2. The wearable device (C, C1, C2, C3) of claim 1, wherein the data storage (21) is configured to store information sensed by the snoring sensor, and the data transmitter (22) is configured to remotely transmit the sensed information stored in the data storage (21).

3. The wearable device (C, C1, C2, C3) of claim 1, the snoring sensor is configured to sense the oxygen saturation occurring when the patient breathes or a sound occurring when the patient snores.

## Patentansprüche

1. Am Zahn zu befestigende tragbare Vorrichtung (C, C1, C2, C3) mit einem Zahnaufsatz (P1, P2, P3), der einer Form eines Zahns entspricht, wobei die tragbare Vorrichtung (C, C1, C2, C3) Folgendes umfasst:
einen Sensorkopf (11), der konfiguriert ist, um mit einer Oberfläche des Zahnaufsatzes (P1, P2, P3) zu koppeln, um innerhalb der Mundhöhle freigelegt zu werden und eine Temperatur eines Patienten zu erfassen;
wobei ein Zeitmesser konfiguriert ist, um basierend auf der am Sensorkopf (11) erfassten Temperatur eine Zeitspanne zu messen, in der der Zahnaufsatz (P1, P2, P3) an dem Zahn befestigt ist;
wobei dem Sensorkopf (11) ein Thermoelement (11a) vorgesehen ist, das Thermoelement (11a) Elektrizität erzeugt und die erzeugte Elektrizität nur dann an den Zeitmesser liefert, wenn die am Sensorkopf (11) erfasste Temperatur zwischen 35 °C und 38 °C liegt;
wobei das Zeitmesser die Zeit nur als Reaktion auf den Empfang der vom Thermoelement (11a) gelieferten Elektrizität misst und akkumuliert; und
wobei die tragbare Vorrichtung (C, C1, C2, C3) ferner einen Sensorchip (12) umfasst, der konfiguriert ist, um mit dem Sensorkopf (11) zu koppeln, an dem Zahnaufsatz (P1, P2, P3) befestigt zu werden und dem Sensorkopf (11) Elektrizität zuzuführen;
wobei die tragbare Vorrichtung (C, C1, C2, C3) eine Kommunikationssteuerung (20) umfasst, die dem Zahnaufsatz (P1, P2, P3) vorgesehen und konfiguriert ist, um am Sensorkopf (11) erfasste Temperaturinformationen des Patienten zu speichern und um die gespeicherten Temperaturinformationen zu übertragen,
wobei die Kommunikationssteuerung einen Datenspeicher (21), der konfiguriert ist, um die am Sensorkopf (11) erfassten Temperaturinformationen des Patienten als Daten zu speichern; und einen Datensender (22) umfasst, der konfiguriert ist, um die in dem Datenspeicher (21) gespeicherten Daten aus der Ferne zu übertragen,
wobei die tragbare Vorrichtung (C, C1, C2, C3) ferner Folgendes umfasst: einen Schnarchsensor, der konfiguriert ist, um mit einer Oberfläche des Zahnaufsatzes (P1, P2, P3) zu koppeln und mindestens eines von einer Vibration, einer Sauerstoffsättigung und einem Geräusch durch Schnarchen zu spüren,
wobei der Sensorchip (12) dazu konfiguriert ist, mit dem Schnarchsensor zu koppeln und dem Schnarchsensor Elektrizität zuzuführen; und die Kommunikationssteuerung dazu konfiguriert ist, mindestens eines von einer Vibration, einer beim Atmen auftretenden Sauerstoffsättigung und einem am Schnarchsensor erfassten Schnarchgeräusch zu speichern und das erfasste mindestens eines von einer Vibration, einer beim Atmen auftretenden Sauerstoffsättigung und einem Schnarchgeräusch zu speichern.

2. Tragbare Vorrichtung (C, C1, C2, C3) nach Anspruch 1, wobei der Datenspeicher (21) konfiguriert ist, um von dem Schnarchsensor erfasste Informationen zu speichern, und der Datensender (22) konfiguriert ist, um die erfassten Informationen, die in dem Datenspeicher (21) gespeichert sind, aus der Ferne zu übertragen.

3. Tragbare Vorrichtung (C, C1, C2, C3) nach Anspruch 1, wobei der Schnarchsensor konfiguriert ist, um die Sauerstoffsättigung, die auftritt, wenn der Patient atmet, oder ein Geräusch, das auftritt, wenn der Patient schnarcht, zu erfassen.

## Revendications

1. Dispositif portable fixé à une dent (C, Cl, C2, C3) ayant une attache dentaire (P1, P2, P3) correspondant à une forme d'une dent, le dispositif portable (C, C1, C2, C3) comprenant :
une tête de capteur (11) configurée pour se coupler à une surface de l'attache dentaire (P1, P2, P3), pour être exposée à l'intérieur de la cavité buccale, et pour détecter une température d'un patient ;
dans lequel un mesureur de temps est configuré pour mesurer une quantité de temps pendant laquelle l'attache dentaire (P1, P2, P3) est fixée à la dent, sur la base de la température détectée au niveau de la tête de capteur (11) ;
dans lequel un thermo-élément (11a) est fourni à la tête de capteur (11), le thermo-élément (11a) génère de l'électricité et fournit l'électricité générée au mesureur de temps uniquement lorsque la température détectée au niveau de la tête de capteur (11) est comprise entre 35 °C et 38 °C ;
dans lequel le mesureur de temps mesure et accumule la quantité de temps uniquement en réponse à la réception de l'électricité fournie par le thermo-élément (11a); et
dans lequel le dispositif portable (C, C1, C2, C3) comprend en outre une puce de capteur (12) configurée pour se coupler avec la tête de capteur (11), pour se fixer à l'attache dentaire (P1, P2, P3), et pour fournir de l'électricité à la tête de capteur (11),
dans lequel le dispositif portable (C, C1, C2, C3) comprend un contrôleur de communication (20) fourni à l'attache dentaire (P1, P2, P3), et configuré pour stocker des informations de température du patient détectées au niveau de la tête de capteur (11), et pour transmettre les informations de température stockées,
dans lequel le contrôleur de communication comprend une mémoire de données (21) configurée pour stocker les informations de température du patient détectées au niveau de la tête de capteur (11) en tant que données ; et un émetteur de données (22) configuré pour transmettre à distance les données stockées dans la mémoire de données (21),
le dispositif portable (C, C1, C2, C3) comprenant en outre : un capteur de ronflement configuré pour se coupler à une surface de l'attache dentaire (P1, P2, P3), et pour détecter au moins l'un parmi une vibration, une saturation en oxygène et un son de ronflement,
dans lequel la puce de capteur (12) est configurée pour se coupler au capteur de ronflement et pour fournir de l'électricité au capteur de ronflement ; et le contrôleur de communication est configuré pour stocker au moins l'un parmi une vibration, une saturation en oxygène se produisant lors de la respiration et un son de ronflement détecté au niveau du capteur de ronflement, et pour stocker au moins l'un parmi une vibration, une saturation en oxygène se produisant lors de la respiration et un son de ronflement.

2. Dispositif portable (C, C1, C2, C3) selon la revendication 1, dans lequel la mémoire de données (21) est configurée pour stocker des informations détectées par le capteur de ronflement, et l'émetteur de données (22) est configuré pour transmettre à distance les informations détectées stockées dans la mémoire de données (21).

3. Dispositif portable (C, C1, C2, C3) selon la revendication 1, le capteur de ronflement est configuré pour détecter la saturation en oxygène se produisant lorsque le patient respire ou un son se produisant lorsque le patient ronfle.
